# EUROPEAN PATENT SPECIFICATION

(11) **EP 3 100 682 B1**
(45) Date of publication and mention of the grant of the patent: **13.05.2020**
(21) Application number: 16172805.0
(22) Date of filing: 03.06.2016
(51) Int. Cl.: A61B 6/12, A61B 6/00, G06T 7/00, A61B 90/00, A61B 5/06, G06T 7/33, A61B 5/00, A61B 5/055, A61B 6/03, A61B 34/20

(54) **REGISTRATION OF CORONARY SINUS CATHETER IMAGE**
REGISTRIERUNG VON KORONARSINUSKATHETERBILDERN
ENREGISTREMENT D'IMAGE DE CATHÉTER DANS LE SINUS CORONAIRE

(30) Priority: 04.06.2015 US 201514730386
(43) Date of publication of application: 07.12.2016
(73) Proprietor: Biosense Webster (Israel) Ltd., 2066717 Yokneam (IL)
(72) Inventor: BAR-TAL, Meir, 3224009 Haifa (IL)
(74) Representative: Carpmaels & Ransford LLP

(56) References cited:
- US-A1- 2005 080 328
- US-A1- 2006 079 759
- US-A1- 2006 116 575
- US-A1- 2008 219 536

## Description

### BACKGROUND OF THE INVENTION

### Field of the Invention

This invention relates to cardiac physiology. More particularly, this invention relates to the evaluation of electrical propagation in the heart.

### Description of the Related Art

The meanings of certain acronyms and abbreviations used herein are given in Table 1.

**Table 1 - Acronyms and Abbreviations**

| | |
|---|---|
| CS | Coronary Sinus |
| LAO | Left Anterior Oblique |
| RAO | Right Anterior Oblique |
| CT | Computed Tomography |
| MRI | Magnetic Resonance Image |

Cardiac arrhythmias such as atrial fibrillation are an important cause of morbidity and death. Commonly assigned U.S. Patent No. 5,546,951, and U.S. Patent No. 6,690,963, both issued to Ben Haim, and PCT application WO 96/05768 disclose methods for sensing an electrical property of heart tissue, for example, local activation time, as a function of the precise location within the heart. Data are acquired with one or more catheters having electrical and location sensors in their distal tips, which are advanced into the heart. Methods of creating a map of the electrical activity of the heart based on these data are disclosed in commonly assigned U.S. Patent No. 6,226,542, and U.S. Patent No. 6,301,496, both issued to Reisfeld. As indicated in these patents, location and electrical activity is typically initially measured on about 10 to about 20 points on the interior surface of the heart. These data points are then generally sufficient to generate a preliminary reconstruction or map of the cardiac surface. The preliminary map is often combined with data taken at additional points in order to generate a more comprehensive map of the heart's electrical activity. Indeed, in clinical settings, it is not uncommon to accumulate data at 100 or more sites to generate a detailed, comprehensive map of heart chamber electrical activity. The generated detailed map may then serve as the basis for deciding on a therapeutic course of action, for example, tissue ablation, to alter the propagation of the heart's electrical activity and to restore normal heart rhythm.

Catheters containing position sensors may be used to determine the trajectory of points on the cardiac surface. These trajectories may be used to infer motion characteristics such as the contractility of the tissue. As disclosed in U.S. Patent No. 5,738,096, issued to Ben Haim, maps depicting such motion characteristics may be constructed when the trajectory information is sampled at a sufficient number of points in the heart.

Electrical activity at a point in the heart is typically measured by advancing a multiple-electrode catheter to measure electrical activity at multiple points in the heart chamber simultaneously. A record derived from time varying electrical potentials as measured by one or more electrodes is known as an electrogram. Electrograms may be measured by unipolar or bipolar leads, and are used, e.g., to determine onset of electrical propagation at a point, known as local activation time.

Currently, large amounts of anatomical and functional data are gathered during catheter-based cardiac procedures. Maintaining alignment of this data with the actual position of the patient's heart and understanding the relationship of the catheter to anatomic structures are both important to the success of the procedure. In one approach the catheter is registered to images taken by another modality.

An example of this approach is proposed in U.S. Patent No. 7,720,520 to Willis. A reference catheter or reference element is placed in contact with the anatomical body. A physical structure within a navigational coordinate system is located using the reference elements or reference catheter. An image reference within an image coordinate corresponding to the physical structure is located. Location of the image reference can be accomplished, e.g., by displaying the medical image and electronically marking the displayed image reference, or by automatically locating image data corresponding to the image reference. The navigational and image coordinate systems are then registered based on the location of the physical structure within the navigational coordinate system and the location of the image reference within the image coordinate system, which allows graphical information to be merged with the medical image data.

US2008/0219536A1 describes a method for registering images of multiple modalities including acquiring a first image of a subject using a first modality, and a second image of the subject using a second modality; and registering the first and second images based on anatomical structures observable in the first image and a foreign object proximate to the anatomical structure observable in the second image.

### SUMMARY OF THE INVENTION

The invention is defined by independent claim 1. According to disclosed embodiments of the invention, a reconstruction of the heart is prepared prior to catheterization from cardiac image data, such as CT or MRI data. The reconstruction takes into consideration motion of the cardiac structures such as the coronary sinus due to cardiac and patient motion. Using the reconstruction, the coronary sinus is catheterized and reconstructed using fluoroscopic image data. The reconstruction of the coronary sinus is placed in registration with the reconstruction of the heart, so that the resulting image has a coordinate space consistent with that of a functional electroanatomic images of the heart that may be displayed or generated, for example a CARTO map. Cardiac structures of interest may then be identified on the functional electroanatomic image by an operator.

There is provided a method, which is carried out by importing image data of a heart of a living subject into an image-processing computer system, representing the image data as a first model of the heart and the coronary sinus in a first coordinate space, and introducing a probe into the coronary sinus. Thereafter, the method is further carried out acquiring fluoroscopic image data of the probe, using the fluoroscopic image data to prepare a second model of the coronary sinus in a second coordinate space, and transforming the first model into the second coordinate space by placing the coronary sinus of the second model in registration with the coronary sinus of the first model.

The image data is obtained by computed tomography or magnetic resonance imaging of the heart.

According to an aspect of the method, the first model is a 3-dimensional model.

According to still another aspect of the method, preparing a second model includes reconstructing a 2-dimensional path of the probe.

According to an additional aspect of the method, preparing a second model includes estimating a 3-dimensional path of the probe from the 2-dimensional path.

Another aspect of the method includes locating a cardiac structure in the transformed first model in the second coordinate space.

There is further provided according to embodiments of the invention an apparatus, including a cardiac catheter adapted for introduction into a coronary sinus of a heart of a living subject, a display, and a processor. The processor is cooperative with a fluoroscopic imaging device for performing a method, which is carried out by importing image data of the heart into an image-processing computer system, representing the image data as a first model of the heart and the coronary sinus in a first coordinate space, and introducing a probe into the coronary sinus. Thereafter, the method is further carried out acquiring fluoroscopic image data of the probe, using the fluoroscopic image data to prepare a second model of the coronary sinus in a second coordinate space, and transforming the first model into the second coordinate space by placing the coronary sinus of the second model in registration with the coronary sinus of the first mode.

### BRIEF DESCRIPTION OF THE SEVERAL VIEWS OF THE DRAWINGS

For a better understanding of the present invention, reference is made to the detailed description of the invention, by way of example, which is to be read in conjunction with the following drawings, wherein like elements are given like reference numerals, and wherein:
Fig. 1 is a pictorial illustration of a system for performing cardiac catheterization procedures, which is constructed and operative in accordance with an embodiment of the invention;
Fig. 2 is a flow-chart of a method of registration of cardiac images using a coronary sinus catheter; and
Fig. 3 is a composite image illustrating a stage in the process of coronary sinus reconstruction as applied to a 3-dimensional model of the heart.

### DETAILED DESCRIPTION OF THE INVENTION

In the following description, numerous specific details are set forth in order to provide a thorough understanding of the various principles of the present invention. It will be apparent to one skilled in the art, however, that not all these details are necessarily needed for practicing the present invention. In this instance, well-known circuits, control logic, and the details of computer program instructions for conventional algorithms and processes have not been shown in detail in order not to obscure the general concepts unnecessarily.

Aspects of the present invention may be embodied in software programming code, which is typically maintained in permanent storage, such as a computer readable medium. In a client/server environment, such software programming code may be stored on a client or a server. The software programming code may be embodied on any of a variety of known non-transitory media for use with a data processing system, such as USB memory, hard drive, electronic media or CD-ROM. The code may be distributed on such media, or may be distributed to users from the memory or storage of one computer system over a network of some type to storage devices on other computer systems for use by users of such other systems.

Embodiments of the invention enable identifying cardiac structures during the medical procedure. When the position of the coronary sinus is accurately known based on fluoroscopy of a coronary sinus catheter, its position can be related to the position of other portions of the heart provided there is a common coordinate system to locate the other portions, the embodiments of the invention place the coronary sinus, as determined from fluoroscopy, in registration with a 3-dimensional model of the heart prepared using another imaging modality.

### System Overview

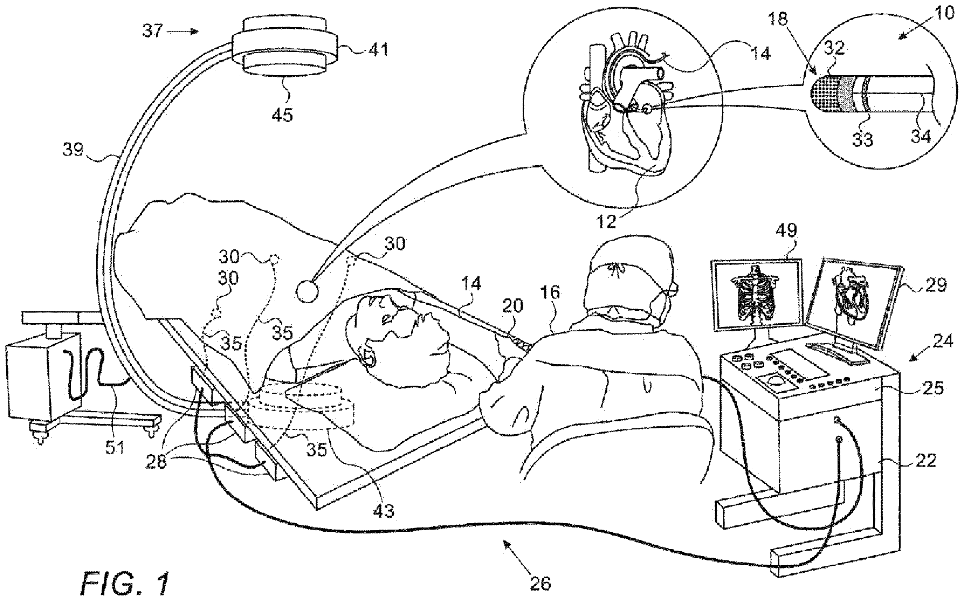

Turning now to the drawings, reference is initially made to Fig. 1, which is a pictorial illustration of a system 10 for performing catheterization procedures on a heart 12 of a living subject, which is constructed and operative in accordance with a disclosed embodiment of the invention. The system comprises a catheter 14, which is percutaneously inserted by an operator 16 through the patient's vascular system into a chamber or vascular structure of the heart 12. The operator 16, who is typically a physician, brings the catheter's distal tip 18 into contact with the heart wall at an ablation target site. Electrical activation maps, anatomic positional information, i.e., of the distal portion of the catheter, and other functional images may then be prepared using a processor 23 located in a console 24, according to the methods disclosed in U.S. Patent Nos. 6,226,542, and 6,301,496, and in commonly assigned U.S. Patent No. 6,892,091. One commercial product embodying elements of the system 10 is available as the CARTO® 3 System, available from Biosense Webster, Inc., 3333 Diamond Canyon Road, Diamond Bar, CA 91765, which is capable of producing electroanatomic maps of the heart as required for the ablation. This system may be modified by those skilled in the art to embody the principles of the invention described herein.

Areas determined to be abnormal, for example by evaluation of the electrical activation maps, can be ablated by application of thermal energy, e.g., by passage of radiofrequency electrical current through wires in the catheter to one or more electrodes at the distal tip 18, which apply the radiofrequency energy to the myocardium. The energy is absorbed in the tissue, heating (or cooling) it to a point (typically about 60°C) at which it permanently loses its electrical excitability. When successful, this procedure creates non-conducting lesions in the cardiac tissue, which disrupt the abnormal electrical pathway causing the
arrhythmia. The principles of the invention can be applied to different heart chambers to treat many different cardiac arrhythmias.

The catheter 14 typically comprises a handle 20, having suitable controls on the handle to enable the operator 16 to steer, position and orient the distal end of the catheter as desired for the ablation. To aid the operator 16, the distal portion of the catheter 14 contains position sensors (not shown) that provide signals to a positioning processor 22, located in the console 24.

Ablation energy and electrical signals can be conveyed to and from the heart 12 through the catheter tip and/or one or more ablation electrodes 32 located at or near the distal tip 18 via cable 34 to the console 24. Pacing signals and other control signals may be conveyed from the console 24 through the cable 34 and the electrodes 32 to the heart 12. Sensing electrodes 33, also connected to the console 24 are disposed between the ablation electrodes 32 and have connections to the cable 34.

Wire connections 35 link the console 24 with body surface electrodes 30 and other components of a positioning sub-system. The electrodes 32 and the body surface electrodes 30 may be used to measure tissue impedance at the ablation site as taught in U.S. Patent No. 7,536,218, issued to Govari et al.*.* A temperature sensor (not shown), typically a thermocouple or thermistor, may be mounted on or near each of the electrodes 32.

The console 24 typically contains one or more ablation power generators 25. The catheter 14 may be adapted to conduct ablative energy to the heart using any known ablation technique, e.g., radiofrequency energy, ultrasound energy, freezing technique and laser-produced light energy. Such methods are disclosed in commonly assigned U.S. Patent Nos. 6,814,733, 6,997,924, and 7,156,816.

The positioning processor 22 is an element of a positioning subsystem in the system 10 that measures location and orientation coordinates of the catheter 14.

In one embodiment, the positioning subsystem comprises a magnetic position tracking arrangement that determines the position and orientation of the catheter 14 by generating magnetic fields in a predefined working volume and sensing these fields at the catheter, using field generating coils 28. The positioning subsystem may employ impedance measurement, as taught, for example in U.S. Patent No. 7,756,576 and in the above-noted U.S. Patent No. 7,536,218.

A fluoroscopic imaging device 37 has a C-arm 39, an x-ray source 41, an image intensifier module 43 and an adjustable collimator 45. A control processor (not shown), which may be located in the console 24, allows an operator to control the operation of the fluoroscopic imaging device 37, for example by setting imaging parameters, and controlling the collimator 45 to adjust the size and position of the field of view. The control processor may communicate with the fluoroscopic imaging device 37 via a cable 51 to enable and disable the x-ray source 41 or restrict its emissions to a desired region of interest by controlling the collimator 45, and to acquire image data from the image intensifier module 43. An optional display monitor 49, linked to the control processor, allows the operator to view images produced by the fluoroscopic imaging device 37. When the display monitor 49 is not included, the fluoroscopic images may be viewed on a monitor 29, either via a split screen or in alternation with other non-fluoroscopic images.

As noted above, the catheter 14 is coupled to the console 24, which enables the operator 16 to observe and regulate the functions of the catheter 14. The processor 23 is typically a computer with appropriate signal processing circuits. The processor 23 is coupled to drive the monitor 29. The signal processing circuits typically receive, amplify, filter and digitize signals from the catheter 14, including signals generated by the above-noted sensors and a plurality of location sensing electrodes (not shown) located distally in the catheter 14. The digitized signals are received and used by the console 24 and the positioning system to compute the position and orientation of the catheter 14 and analyze the electrical signals from the electrodes, and generate desired electroanatomic maps.

Typically, the system 10 includes other elements, which are not shown in the figures for the sake of simplicity. For example, the system 10 may include an electrocardiogram (ECG) monitor, coupled to receive signals from one or more body surface electrodes, to provide an ECG synchronization signal to the console 24. As mentioned above, the system 10 typically also includes a reference position sensor, either on an externally-applied reference patch attached to the exterior of the subject's body, or on an internally-placed catheter, which is inserted into the heart 12 maintained in a fixed position relative to the heart 12. Conventional pumps and lines for circulating liquids through the catheter 14 for cooling the ablation site are provided.

### Operation

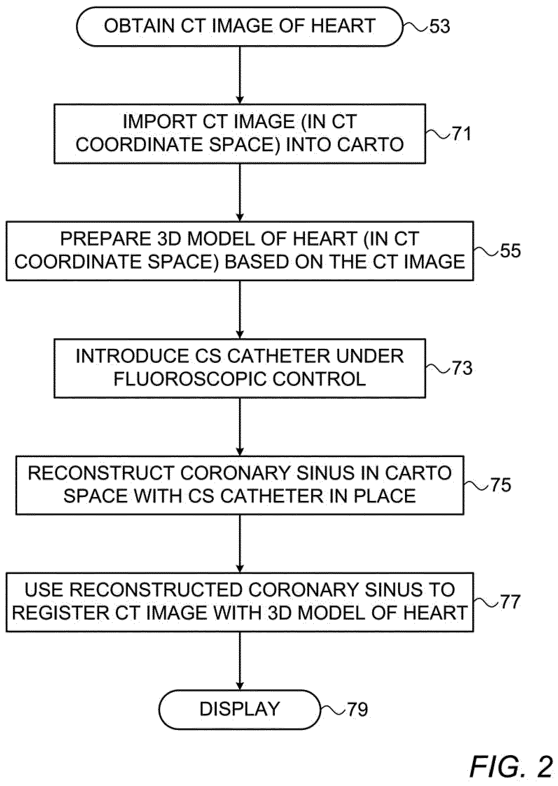

Reference is now made to Fig. 2, which is a flow-chart of a method of registration of cardiac images using a coronary sinus catheter. The process steps are shown in a particular linear sequence for clarity of presentation. However, it will be evident that many of them can be performed in parallel, asynchronously, or in different orders. Those skilled in the art will also appreciate that a process could alternatively be represented as a number of interrelated states or events, e.g., in a state diagram. Moreover, not all illustrated process steps may be required to implement the method.

At initial step 53 a CT (or MRI) image of the heart is obtained. This may be accomplished prior to the current session, but in any case prior to introduction of a cardiac catheter. The image created in this step has a scale and coordinate system that is specific to the image acquisition device employed, which is referred to for convenience as "CT coordinates". The term "CT coordinate space" describes a 3-dimensional space having points described in CT coordinates. The coronary sinus is defined in CT coordinates on the image.

Next, at step 71 the CT image is imported into an image processing computer, e.g., the above-described CARTO 3 system. At this stage, the imported image occupies CT coordinate space. However, images normally produced using the image processing computer have another coordinate system and occupy a different coordinate space. This coordinate system and space are referred to herein for convenience as "CARTO coordinates" and "CARTO coordinate space", respectively. It will be understood that the use of this terminology does not limit the application of the method to the CARTO 3 system. Rather the steps may be performed by many other types of image processing computers.

Next at step 55, using the image processing computer of step 71, a 3-dimensional model of the heart in CT coordinate space is prepared from the CT image. This can be done using the CARTOMERGE™ module, available from Biosense Webster.

Next, at step 73 a coronary sinus catheter is introduced into the coronary sinus under fluoroscopic control.

Next, at step 75, with the coronary sinus catheter in place, the coronary sinus is reconstructed by the image processing computer using the techniques taught in commonly assigned copending Application Nos. 14/621,570 and 14/621,581. Briefly, a 3-dimensional path of the coronary sinus catheter may be estimated using epi-polar geometry or iterative reconstruction of linear segments. In one method of reconstructing the coronary sinus, the patient's heart position is tracked over time. In order to compensate for heart movement, an algorithm reconstructs a path or track of the coronary sinus catheter in three dimensions, based on two 2-dimensional fluoroscopic images acquired before and after a movement, and synchronized in the cardiorespiratory cycle. A transformation between the two reconstructed catheters is computed and used to align the data. The reconstructed coronary sinus is motion-compensated, and exists in CARTO coordinate space.

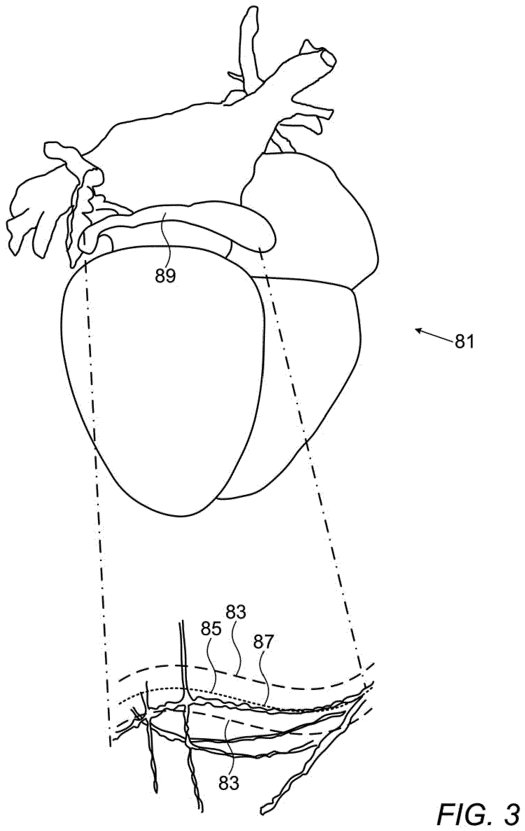

Reference is now made to Fig. 3, which is a composite image illustrating a stage in the process of coronary sinus reconstruction as applied to a 3-dimensional model 81 of the heart as described in step 55. In the lower portion of the figure, taken from a fluoroscopic frame, a corridor 83 is outlined and sampled around a CS catheter path 85 (represented by a broken line). The catheter path 85 was defined from previous frame and may have been annotated by a human operator. The divergence of CS catheter 87 from the catheter path 85 is compensated by suitable transformations, as explained in further detail in the above-noted Application Nos. 14/621,570 and 14/621,581,

Referring again to Fig. 2 together with Fig. 3, at step 77 the reconstructed coronary sinus is placed in registration with the 3-dimensional model that was prepared in step 55 by aligning coronary sinus 89 in the model 81 with the transformed catheter path 85 obtained from analysis of the fluoroscopic image. It will be recalled that the 3-dimensional model 81 exists in CT coordinate space. The model 81 is now transformed into CARTO coordinate space. The CARTOMERGE™ image integration module, available from Biosense Webster, is suitable for performing the image registration and coordinate transformation. Since the position or the coronary sinus is closely related to the position of other portions of the heart, the remainder of the heart on the model 81 will be in registration with other cardiac images generated and displayed by the image processing computer.

Then in final step 79, the coronary sinus and the other cardiac structures can now be accurately located on the image processing computer display in CARTO coordinate space.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

## Claims

1. An apparatus, comprising:
a cardiac catheter (14) adapted for introduction into a coronary sinus of a heart of a living subject;
a display (29);
a fluoroscopic imaging device (37); and
a processor (23), which is cooperative with the fluoroscopic imaging device (37) and is configured for performing the steps of:
importing image data of the heart (71) obtained prior to introduction of the cardiac catheter;
representing the image data as a first model of the heart in a first coordinate space, the first model comprising the coronary sinus (55);
thereafter acquiring a first fluoroscopic image data of the cardiac catheter (14) in the coronary sinus of the heart of the living subject; and then
acquiring a second fluoroscopic image data of the cardiac catheter (14) in the coronary sinus of the heart of the living subject, wherein the first and second fluoroscopic image data are acquired before and after a movement of the heart; wherein the first and second fluoroscopic image data are synchronised in a cardiorespiratory cycle of the living subject and the processor is further configured for performing the steps of:
using the first and second fluoroscopic image data of the cardiac catheter (14) to prepare a second model of the coronary sinus in a second coordinate space (75); and
transforming the first model into the second coordinate space by placing the coronary sinus of the second model in registration with the coronary sinus of the first model (77).

2. The apparatus according to claim 1, wherein the image data is obtained by computed tomography of the heart.

3. The apparatus according to claim 1, wherein the image data is obtained from magnetic resonance imaging of the heart.

4. The apparatus according to claim 1, wherein the first model is a 3-dimensional model.

5. The apparatus according to claim 1, wherein preparing a second model comprises reconstructing a 2-dimensional path of the cardiac catheter.

6. The apparatus according to claim 5, wherein preparing a second model comprises estimating a 3-dimensional path of the cardiac catheter from the 2-dimensional path.

7. The apparatus according to claim 1, further comprising locating a cardiac structure in the transformed first model in the second coordinate space.

## Patentansprüche

1. Vorrichtung, umfassend:
einen Herzkatheter (14), der zur Einführung in einen Koronarsinus eines Herzens eines lebenden Subjekts angepasst ist;
eine Anzeige (29);
eine Durchleuchtungsbildgebungsvorrichtung (37); und
einen Prozessor (23), der mit der Durchleuchtungsbildgebungsvorrichtung (37) zusammenwirkt und ausgelegt ist zum Durchführen der Schritte:
Importieren von Bilddaten des Herzens (71), die vor der Einführung des Herzkatheters erhalten wurden;
Darstellen der Bilddaten als ein erstes Modell des Herzens in einem ersten Koordinatenraum, wobei das erste Modell den Koronarsinus umfasst (55);
danach Erfassen von ersten Durchleuchtungsbilddaten des Herzkatheters (14) in dem Koronarsinus des Herzens des lebenden Subjekts; und dann
Erfassen von zweiten Durchleuchtungsbilddaten des Herzkatheters (14) in dem Koronarsinus des Herzens des lebenden Subjekts, wobei die ersten und zweiten Durchleuchtungsbilddaten vor und nach einer Bewegung des Herzens erfasst werden; wobei die ersten und zweiten Durchleuchtungsbilddaten in einem kardiorespiratorischen System des lebenden Subjekts synchronisiert werden und der Prozessor ferner ausgelegt ist zum Durchführen der Schritte:
Verwenden der ersten und der zweiten Durchleuchtungsbilddaten des Herzkatheters (14) zum Erstellen eines zweiten Modells des Koronarsinus in einem zweiten Koordinatenraum (75); und
Transformieren des ersten Modells in den zweiten Koordinatenraum durch Platzieren des Koronarsinus des zweiten Modells in Deckung mit dem Koronarsinus des ersten Modells (77).

2. Vorrichtung nach Anspruch 1, wobei die Bilddaten mithilfe von Computertomographie des Herzens erhalten werden.

3. Vorrichtung nach Anspruch 1, wobei die Bilddaten mithilfe von Magnetresonanzbildgebung des Herzens erhalten werden.

4. Vorrichtung nach Anspruch 1, wobei das erste Modell ein 3-dimensionales Modell ist.

5. Vorrichtung nach Anspruch 1, wobei das Erstellen eines zweiten Modells das Rekonstruieren eines 2-dimensionalen Weges des Herzkatheters umfasst.

6. Vorrichtung nach Anspruch 5, wobei das Erstellen eines zweiten Modells das Schätzen eines 3-dimensionalen Weges des Herzkatheters aus dem 2-dimensionalen Weg umfasst.

7. Vorrichtung nach Anspruch 1, ferner umfassend das Lokalisieren einer Herzstruktur in dem transformierten ersten Modell in dem zweiten Koordinatenraum.

## Revendications

1. Appareil, comprenant :
un cathéter cardiaque (14) conçu pour une introduction dans un sinus coronaire d'un cœur d'un sujet vivant ;
un affichage (29) ;
un dispositif d'imagerie fluoroscopique (37) ; et
un processeur (23), qui coopère avec le dispositif d'imagerie fluoroscopique (37) et qui est conçu pour réaliser les étapes consistant à :
importer des données d'image du cœur (71) obtenues avant l'introduction du cathéter cardiaque ;
représenter les données d'image sous la forme d'un premier modèle du cœur dans un premier espace de coordonnées, le premier modèle comprenant le sinus coronaire (55) ;
après cela, acquérir des premières données d'image fluoroscopique du cathéter cardiaque (14) dans le sinus coronaire du cœur du sujet vivant ; puis
acquérir des secondes données d'image fluoroscopique du cathéter cardiaque (14) dans le sinus coronaire du cœur du sujet vivant, les premières et secondes données d'image fluoroscopique étant acquises avant et après un mouvement du cœur ; dans lequel les premières et secondes données d'image fluoroscopique sont synchronisées dans un cycle cardiorespiratoire du sujet vivant et le processeur est en outre configuré pour réaliser les étapes consistant à :
utiliser les première et seconde données d'image fluoroscopique du cathéter cardiaque (14) pour préparer un second modèle du sinus coronaire dans un second espace de coordonnées (75) ; et
transformer le premier modèle dans le second espace de coordonnées en plaçant le sinus coronaire du second modèle en alignement sur le sinus coronaire du premier modèle (77).

2. Appareil selon la revendication 1, dans lequel les données d'image sont obtenues par une tomodensitométrie du cœur.

3. Appareil selon la revendication 1, dans lequel les données d'image sont obtenues à partir d'une imagerie par résonance magnétique du cœur.

4. Appareil selon la revendication 1, dans lequel le premier modèle est un modèle tridimensionnel.

5. Appareil selon la revendication 1, dans lequel la préparation d'un second modèle consiste à reconstruire un trajet bidimensionnel du cathéter cardiaque.

6. Appareil selon la revendication 5, dans lequel la préparation d'un second modèle consiste à estimer un trajet tridimensionnel du cathéter cardiaque à partir du trajet bidimensionnel.

7. Appareil selon la revendication 1, consistant en outre à localiser une structure cardiaque dans le premier modèle transformé dans le second espace de coordonnées.
